(19) Europäisches Patentamt
European Patent Office
Office européen des brevets

(11) EP 2 545 902 A2

# (12) EUROPÄISCHE PATENTANMELDUNG

(43) Veröffentlichungstag:
**16.01.2013 Patentblatt 2013/03**

(21) Anmeldenummer: **12174550.9**

(22) Anmeldetag: **02.07.2012**

(51) Int Cl.:
***A61K 8/35*** *(2006.01)* ***A61K 8/37*** *(2006.01)*
***A61K 8/40*** *(2006.01)* ***A61K 8/46*** *(2006.01)*
***A61K 8/49*** *(2006.01)* ***A61Q 17/04*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **15.07.2011 DE 102011079256**

(71) Anmelder: **Henkel AG & Co. KGaA**
**40589 Düsseldorf (DE)**

(72) Erfinder:
- **Weiß, Thomas**
  **40591 Düsseldorf (DE)**
- **Dickhof, Susanne**
  **41748 Viersen (DE)**

## (54) Sonnenschutzzusammensetzungen mit verbessertem antioxidativen Potenzial zum Schutz der Haut vor IR-Strahlung

(57) Gegenstand der vorliegenden Anmeldung sind kosmetische Zusammensetzung, enthaltend 2-Cyano-3,3-phenylzimtsäure-2-ethylhexylester, 4-(tert.-Butyl)-4'-methoxydibenzoylmethan, 3,3,5-Trimethylcyclohexylsalicylat (Homosalate), mindestens einen wasserlöslichen organischen UV-Filter, mindestens eine Substanz, ausgewählt aus 6,7-disubstituierten 2,2-Dialkylchromanen oder - chromenen der allgemeinen Formeln (I) oder (II),

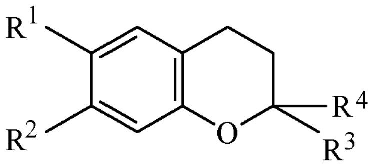

(I)

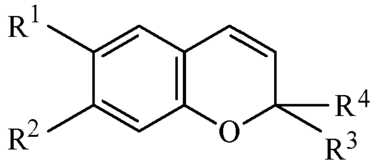

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere 3,4-Dihydro-7-methoxy-2,2-dimethyl-2H-1-benzopyran-6-ol, und wobei die Zusammensetzung kein Polysilicone-15 enthält, sowie deren Verwendung zum Schutz der Haut und des Haars vor UV-Strahlung und vor IR-Strahlung.

EP 2 545 902 A2

**Beschreibung**

Die vorliegende Erfindung betrifft kosmetische und dermatologische Zusammensetzungen zum Schutz von Haut und Haar gegen UV-Strahlung und IR-Strahlung, die eine hohe antioxidative Wirkung aufweisen. Die Haut schädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.

Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

Neuere wissenschaftliche Untersuchungen haben gezeigt, dass auch IR-Strahlung zur Schädigung von Hautzellen führen kann, obwohl diese Strahlungsart weniger energiereich ist als UV-Strahlung. Laut diesen Studien führt die Bestrahlung der Haut mit IR-Strahlung im Wellenlängenbereich von 760-1400 nm zu einer Aktivierung des MAP-Kinase-Signalwegs (mitogen-activated protein-Kinase, MAPK) und zu einer vermehrten Expression der Matrixmetalloproteinase MMP-1, einer Kollagenase, in den Fibroblasten (J. Krutmann, Hautarzt 2003, 54, 809 - 817). Mit der gesteigerten Expression von MMP-1 geht ein verstärkter Abbau des Kollagens einher, das einen wesentlichen Bestandteil der extracellulären Matrix (ECM) in der Dermis darstellt und dessen Verlust eine Erschlaffung der Haut und die Bildung von Fältchen und Falten bewirkt.

Die Verbesserung des Erscheinungsbildes alternder Haut ist ein wesentliches Ziel kosmetischer Antiage-Pflegeprodukte. Im Stand der Technik sind bereits zahlreiche Wirkstoffe bekannt, die der Expression von MMP-1 entgegenwirken und bei regelmäßiger topischer Applikation das Auftreten von Hautalterungserscheinungen, wie Falten und Fältchen, verzögern können. Zum Schutz der Haut vor der schädigenden Wirkung der UV-Strahlung sind bereits gut wirksame Lichtschutzfiltersubstanzen bekannt. Aus EP 1591105 A1 ist die Verwendung diverser Antioxidantien, darunter Taurin, zum Schutz der Haut vor IR-Strahlung bekannt. Dennoch besteht ein ständiger Bedarf an weiteren Wirkstoffen und Wirkstoffkombinationen zur wirksamen Behandlung von Hautalterungserscheinungen. Besonders interessant sind dabei solche Wirkstoffe und Wirkstoffkombinationen, die möglichst früh in Prozesse, die der Hautalterung zu Grunde liegen, eingreifen und diese stoppen oder zumindest verzögern.

Die Zusammensetzungen des Standes der Technik sind weiter verbesserbar. Insbesondere weisen Zusammensetzungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren. Ein weiterer Nachteil des Standes der Technik ist, dass übliche Lichtschutzformulierungen einen meist klebrigen Film auf der Haut hinterlassen. Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig verbesserte sensorische Eigenschaften aufweisen. Die erfindungsgemäßen Zusammensetzungen sollen ferner gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

DE 102008025576 und EP 2266527 A2 offenbaren Sonnenschutzzusammensetzungen, die einen hohen Schutz gegen die oben genannten verschiedenen nachteiligen Wirkungen der Strahlung im UV-A-und UV-B-Bereich, insbesondere Sonnenstrahlung, bieten, verbesserte sensorische Eigenschaften aufweisen und gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

Der vorliegenden Erfindung lag die Aufgabe zugrunde, alternative Antiage-Zubereitungen bereitzustellen, die die Haut vor den negativen Folgen der IR-Strahlung wie auch vor den negativen Folgen der UV-Strahlung schützen.

Es wurde nun gefunden, dass sich diese Eigenschaften auch durch eine Zusammensetzung erreichen lassen, die eine Kombination aus bestimmten UV-Filtern und bestimmten Antioxidantien enthalten.

Gegenstand der vorliegenden Anmeldung sind kosmetische Zusammensetzung, enthaltend

a) 2-Cyano-3,3-phenylzimtsäure-2-ethylhexylester,
b) 4-(tert.-Butyl)-4'-methoxydibenzoylmethan,
c) 3,3,5-Trimethylcyclohexylsalicylat (Homosalate),
d) mindestens einen wasserlöslichen organischen UV-Filter,
e) mindestens eine Substanz, ausgewählt aus 6,7-disubstituierten 2,2-Dialkylchromanen oder -chromenen der allgemeinen Formeln (I) oder (II),

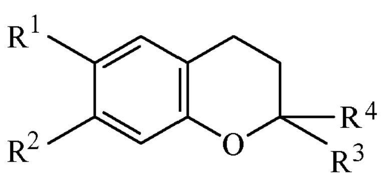

(I)

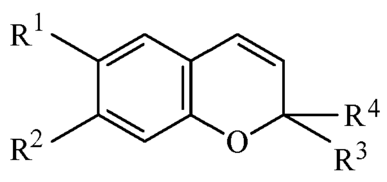

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere 3,4-Dihydro-7-methoxy-2,2-dimethyl-2H-1-benzopyran-6-ol, und wobei die Zusammensetzung kein Polysilicone-15 enthält.

Gegenstand der Erfindung ist ferner die Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung. Gegenstand der Erfindung ist ferner die Verwendung der erfindungsgemäßen vorstehend definierten Zusammensetzungen zum Schutz von Haut und Haar gegen die schädlichen Wirkungen von IR-Strahlung.

Gegenstand der Erfindung ist ferner ein Verfahren zum Schutz von Haut und Haar gegen die schädlichen Wirkungen von IR-Strahlung und/oder gegen die schädlichen Wirkungen von UV-Strahlung, insbesondere UV-A-und UV-B-Strahlung, insbesondere Sonnenstrahlung, bei dem eine erfindungsgemäße oder eine erfindungsgemäß bevorzugte Zusammensetzung auf die Haut oder das Haar aufgetragen wird.

Bevorzugte Ausführungsformen der vorliegenden Erfindung ergeben sich aus den abhängigen Patentansprüchen.

Die erfindungsgemäßen Zusammensetzungen stellen in jeglicher Hinsicht überaus befriedigende Präparate dar, die im Hinblick auf die weiteren Bestandteile nicht auf eine spezielle Auswahl dieser weiteren Bestandteile begrenzt sind. Dementsprechend eignen sie sich ganz besonders, um als Grundlage für Zusammensetzungsformen mit vielfältigen Anwendungszwecken zu dienen. Die erfindungsgemäßen Zusammensetzungen zeigen überraschenderweise synergistisch verbesserte sensorische und kosmetische Eigenschaften, wie beispielsweise die gleichmäßige Verteilbarkeit auf der Haut oder das Einzugsvermögen in die Haut, und zeichnen sich gleichzeitig durch eine hohe Lichtschutzeffektivität bei gleichzeitig hervorragenden Hautpflegedaten aus.

Bevorzugte erfindungsgemäße kosmetische Zusammensetzungen weisen bei 20°C einen pH-Wert im Bereich von 5,7 - 7,3, besonders bevorzugt einen pH-Wert im Bereich von 6,0 bis 6,7, besonders bevorzugt 6,2 bis 6,4, auf.

Erfindungsgemäß bevorzugte kosmetische Zusammensetzungen enthalten, jeweils bezogen auf ihr Gesamtgewicht, bis zu 40 Gew.-%, bevorzugt 17 - 30 Gew.-%, besonders bevorzugt 19 - 27 Gew.-%, außerordentlich bevorzugt 21 - 25 Gew.-%, mindestens einer in der Ölphase oder der Wasserphase gelösten organischen UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate.

Der öllösliche UVB-Filter 2-Cyano-3,3-phenylzimtsäure-2-ethylhexylester (= Ethylhexyl-2-cyano-3,3-diphenylacrylat (INCI: Octocrylene)), ist beispielsweise unter den Handelsnamen Parsol® 340, Eusolex® OCR, Uvinul® N 539 und Neo Heliopano® 303 erhältlich. Octocrylene ist bevorzugt in einer Gesamtmenge von 0,5 bis 15 Gew.-%, besonders bevorzugt 3 bis 13 Gew.-%, insbesondere 5 bis 11 Gew.-% und außerordentlich bevorzugt 8 - 10 Gew.-%, enthalten, jeweils bezogen auf das Gesamtgewicht der erfindungsgemäßen Zusammensetzung.

4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet) ist ein öllöslicher UVA-Lichtschutzfilter.

Bevorzugte erfindungsgemäße Zusammensetzungen enthalten 4-tert-Butyl-4'-Methoxydibenzoylmethan in einer Gesamtmenge von 0,5 bis 10 Gew.-%, bevorzugt 1 bis 8 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

3,3,5-Trimethylcyclohexylsalicylat, auch 3,3,5-Trimethylcyclohexyl-2-hydroxybenzoat oder Homomenthylsalicylat genannt, mit der INCI-Bezeichnung "Homosalate", ist ein öllöslicher UVB-Filter und z.B. als Neo Heliopan HMS von Symrise erhältlich. Bevorzugte erfindungsgemäße Öl-in-Wasser-Emulsionen enthalten 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, jeweils bezogen auf das Gesamtgewicht der erfindungsgemäßen Zusammensetzung. 3,3,5-Trimethylcyclohexylsalicylat hat gegenüber vielen anderen öllöslichen UVB-Filtern, insbesondere gegenüber anderen Salicylsäureester-Filtern, wie 2-Ethylhexylsalicylat oder 4-Isopropylbenzylsalicylat, diverse Vorteile: bei gleicher Konzentration erzielt man einen höheren Lichtschutzfaktor; weiterhin weisen Emulsionen mit diesem Filter im Vergleich zu anderen öllöslichen Salicylsäureester-Filtern, wie 2-Ethylhexylsalicylat, eine bessere Stabilität auf, insbesondere bei längerer Lagerdauer und/oder bei Lagerung bei erhöhten Temperaturen (beispielsweise 45°C). Weiterhin weisen Emulsionen mit diesem Filter im Vergleich zu anderen öllöslichen Salicylsäureester-Filtern, wie 2-Ethylhexylsalicylat oder 4-Isopropylbenzylsalicylat, bessere haptische Eigenschaften auf, insbesondere eine geringere Klebrigkeit und ein weniger stumpfes Hautgefühl.

In einer bevorzugten Ausführungsform sind die erfindungsgemäßen Zusammensetzungen frei von 2-Ethylhexylsalicylat und frei von 4-Isopropylbenzylsalicylat.

Aufgrund des angenehmeren Hautgefühls bevorzugen viele Verbraucher Öl-in-Wasser-Emulsionen gegenüber Wasser-in-Öl-Emulsionen. Da die äußere Wasserphase in solchen Emulsionen häufig einen höheren Gewichtsanteil hat als die dispergierte Öl-/Fettphase, ist es vorteilhaft, die UV-Filtersubstanzen auf beide Phasen zu verteilen und also neben den öllöslichen Filtern 2-Cyano-3,3-phenylzimtsäure-2-ethylhexylester, 4-(tert.-Butyl)-4'-methoxydibenzoylmethan und 3,3,5-Trimethylcyclohexylsalicylat (Homosalate) auch mindestens eine wasserlösliche UV-Filtersubstanz einzuarbeiten.

Die erfindungsgemäßen kosmetischen Zusammensetzungen enthalten demnach mindestens einen wasserlöslichen organischen UV-Filter.

Bevorzugte erfindungsgemäße kosmetische Zusammensetzungen enthalten mindestens einen wasserlöslichen organischen UV-Filter in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung. Derivat der Benzimidazolsulfonsäure

In einer weiteren bevorzugten Ausführungsform der Erfindung umfasst der mindestens eine wasserlösliche organische UV-Filter mindestens ein Derivat der Benzimidazolsulfonsäure.

Erfindungsgemäß bevorzugte Derivate der Benzimidazolsulfonsäure als wasserlösliche organische UV-Filtersubstanz(en) ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-, Lysin-, Arginin-, Histidin- und Ornithin-Salze. Ebenfalls geeignet, wenn auch weniger bevorzugt, sind die Salze dieser Säure mit Amino-$C_1$-$C_6$-Alkanolen und Amino-$C_1$-$C_6$-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS) sowie Mischungen hiervon.

Erfindungsgemäß bevorzugt liegt ein Anteil des mindestens einen Benzimidazolsulfonsäure-Derivats als Salz von mindestens einer basischen Aminosäure, ausgewählt aus Lysin, Arginin, Histidin und Ornithin, vor. Bevorzugt beträgt dieser Anteil mindestens 30 Mol-%, besonders bevorzugt mindestens 50 Mol-%, außerordentlich bevorzugt mindestens 70 Mol-%, jeweils bezogen auf den Gesamtgehalt an Benzimidazolsulfonsäure-Derivat. Eine bevorzugte Aminosäure ist Arginin. Besonders bevorzugt beträgt der Anteil an Arginin-Salz mindestens 30 Mol-%, besonders bevorzugt mindestens 50 Mol.-%, außerordentlich bevorzugt mindestens 70 Mol-%, jeweils bezogen auf den Gesamtgehalt an Benzimidazolsulfonsäure-Derivat.

Neben dem Salz von mindestens einer basischen Aminosäure, ausgewählt aus Lysin, Arginin, Histidin und Ornithin, können weitere Salze des mindestens einen Benzimidazolsulfonsäure-Derivats vorliegen. Als weiteres Salz

bevorzugt sind die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze.

Außerordentlich bevorzugt ist das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte wasserlösliche organische UV-Filtersubstanzen sind die 2-Phenylbenzimidazol-5-sulfonsäure (UV-B-Filter, INCI-Bezeichnung "Phenylbenzimidazole sulfonic acid" (CAS.-Nr. 27503-81-7)) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze, weiterhin die Salze dieser Säure mit mindestens einer basischen Aminosäure, ausgewählt aus Lysin, Arginin, Histidin und Ornithin, insbesondere mit Arginin, insbesondere aber Mischungen der Salze der 2-Phenylbenzimidazol-5-sulfonsäure mit Natriumionen und mit Arginin.

In einer besonders bevorzugten Ausführungsform der Erfindung ist der mindestens eine wasserlösliche organische UV-Filter, der ein Derivat der Benzimidazolsulfonsäure darstellt, ausgewählt aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolamin-Salzen, insbesondere aus Phenylbenzimidazolsulfonsäure und dem Phenylen-1,4-bis-(2-benzimidazyl)-3,3'-5,5'-tetrasulfonsäure-bis-natriumsalz sowie Mischungen hiervon.

In einer weiteren besonders bevorzugten Ausführungsform der Erfindung ist der mindestens eine wasserlösliche organische UV-Filter, der ein Derivat der Benzimidazolsulfonsäure darstellt, ausgewählt aus Phenylen-1,4-bis-(2-benzimidazyl)-3,3'-5,5'-tetrasulfonsäure, 2-Phenylbenzimidazol-5-sulfonsäure und den Salzen dieser Säuren mit mindestens einer basischen Aminosäure, ausgewählt aus Lysin, Arginin, Histidin und Ornithin, insbesondere aus den Salzen der Phenylbenzimidazolsulfonsäure und des Phenylen-1,4-bis-(2-benzimidazyl)-3,3'-5,5'-tetrasulfonsäure-bis-natriumsalzes mit Natrium und mit Arginin als Gegenionen. Diese bevorzugten Salze können in besonders bevorzugter Ausführungsform in Kombination mit Alkali-, Erdalkali-, Ammonium-, Alkylammonium-, Alkanolamin- und Glucammoniumsalzen, bevorzugt mit Natrium-, Kalium-, Trialkylammonium- oder Triethanolamin-Salzen vorliegen.

Bevorzugte erfindungsgemäße Zusammensetzungen enthalten das/die Derivat/e der Benzimidazolsulfonsäure in einer Gesamtmenge von 0,5 bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Diese und die folgenden Gewichtsangaben beziehen sich dabei, unabhängig von der Form, in der das Benzimidazolsulfonsäure-Derivat vorliegt (Salz, freie Säure), auf das Gewicht des Benzimidazolsulfonsäure-Derivats in der Säureform.

Weitere bevorzugte erfindungsgemäße Zusammensetzungen enthalten 2-Phenylbenzimidazol-5-sulfonsäure oder ein Salz hiervon in einer Gesamtmenge von 0,5 bis 8 Gew.-%, bevorzugt 1 bis 6 Gew.-%, besonders bevorzugt 1,5 bis 4 Gew.-% und außerordentlich bevorzugt 2 - 3 Gew.-%, jeweils bezogen auf das Gesamtgewicht der erfindungsgemäßen Zusammensetzung.

Weitere bevorzugte erfindungsgemäße Zusammensetzungen enthalten Phenylen-1,4-bis-(2-benzimidazyl)-3,3'-5,5'-tetrasulfonsäure oder ein Salz hiervon in einer Gesamtmenge von 0,1 bis 10 Gew.-%, bevorzugt 0,5 bis 8 Gew.-%, besonders bevorzugt 0,8 bis 5 Gew.-% und außerordentlich bevorzugt 0,9 - 2 Gew.-%, jeweils bezogen auf das Gesamtgewicht der erfindungsgemäßen Zusammensetzung.

In weiteren bevorzugten erfindungsgemäßen Zusammensetzungen beträgt die Gesamtmenge aller Benzimidazolsulfonsäure-Derivate 0,5 bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung.

Weitere bevorzugte erfindungsgemäße Zusammensetzungen enthalten Phenylen-1,4-bis-(2-benzimidazyl)-3,3'-5,5'-tetrasulfonsäure und 2-Phenylbenzimidazol-5-sulfonsäure oder Salze hiervon in einer Gesamtmenge von 0,5 - 15 Gew.-%, bevorzugt 1 - 10 Gew.-%, besonders bevorzugt 2 - 5 Gew.-% und außerordentlich bevorzugt 3 - 4 Gew.-%, jeweils bezogen auf das Gesamtgewicht der erfindungsgemäßen Zusammensetzung.

Als Inhaltsstoffe) enthalten die erfindungsgemäßen Mittel 6,7-disubstituierte 2,2-Dialkylchromane oder -chromene der allgemeinen Formeln (I) oder (II),

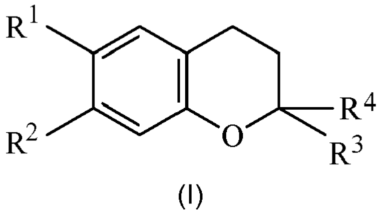

(I)

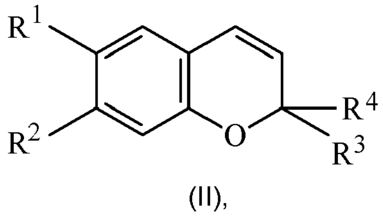

(II),

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen.

Die Substituenten $R^1$ und $R^2$ sind unabhängig voneinander ausgewählt aus einer OH-Gruppe, einer Methoxy-Gruppe und einer $CF_3CH_2O$-Gruppe. In einer bevorzugten Ausführungsform sind $R^1$ und $R^2$ unabhängig voneinander ausgewählt aus einer OH-Gruppe und einer Methoxy-Gruppe. In einer besonders bevorzugten Ausführungsform stellen $R^1$ eine OH-Gruppe und $R^2$ eine Methoxy-Gruppe dar.

Die Substituenten $R^3$ und $R^4$ stellen unabhängig voneinander eine $C_1$-$C_4$-Alkylgruppe dar. Unter $C_1$-$C_4$-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind $R^3$ und $R^4$ unabhängig voneinander ausgewählt aus einer Methyl-, Ethyl-, n-Propyl-, Isopropyl- und einer n-Butyl-Gruppe. Besonders bevorzugt stellen $R^3$ und $R^4$ unabhängig voneinander eine Methyl- oder Ethylgruppe dar. Außerordentlich bevorzugt ist, dass $R^3$ und $R^4$ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Erfindungsgemäß bevorzugte Zusammensetzungen enthalten - bezogen auf ihr Gewicht - mindestens ein 6,7-disubstituiertes 2,2-Dialkylchroman oder -chromen der allgemeinen Formeln (I) oder (II) in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht - eine Mischung aus der oxidierten und der reduzierten Form von 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%. Weitere erfindungsgemäß bevorzugte Zusammensetzungen enthalten mindestens ein Salz von $C_{12-20}$-Alkylphosphaten, das als anionischer Öl-in-Wasser-Emulgator einen ersten Teil eines erfindungsgemäß bevorzugten O/W-Emulgatorsystems bildet. Bevorzugte Salze von $C_{12-20}$-Alkylphosphaten sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.

Weitere erfindungsgemäß bevorzugte Salze von $C_{12-20}$-Alkylphosphaten, die als anionischer Öl-in-Wasser-Emulgator einen ersten Teil des erfindungsgemäß bevorzugten O/W-Emulgatorsystems bilden, sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.

Besonders bevorzugt sind Mischungen aus Mono-$C_{12-20}$-Alkylphosphaten und Di-$C_{12-20}$-Alkylphosphaten. Außerordentlich bevorzugt sind Mischungen aus Dikaliummonocetylphosphat und Kaliumdicetylphosphat.

Erfindungsgemäß bevorzugte $C_{14-20}$-Mono- oder Diacylglyceride, die den zweiten Teil des erfindungsgemäß bevorzugten O/W-Emulgatorsystems bilden, sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid. Weitere erfindungsgemäß bevorzugte $C_{14-20}$-Mono- oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Das mindestens eine $C_{14-20}$-Mono- oder Diacylglycerid ist in erfindungsgemäß bevorzugten Zusammensetzungen in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung, enthalten.

Bevorzugte erfindungsgemäße Zusammensetzungen enthalten mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon, in einer Gesamtmenge

von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung.

Weitere bevorzugte erfindungsgemäße Zusammensetzungen enthalten als $C_{14-20}$-Mono- oder Diacylglycerid-mischung gehärtete Palmölglyceride in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung.

Weitere bevorzugte erfindungsgemäße Zusammensetzungen enthalten, jeweils bezogen auf das Gesamtgewicht der Zusammensetzung, eine Mischung aus Dikaliummonocetylphosphat und Kaliumdicetylphosphat in einer Gesamtmenge von 0,5 - 2,0 Gew.-%, bevorzugt 0,8 - 1,8 Gew.-%, besonders bevorzugt 1,2 - 1,5 Gew.-%, weiterhin in einer Gesamtmenge von 0,3 - 1,7 Gew.-%, bevorzugt 0,6 - 1,4 Gew.-%, besonders bevorzugt 0,9 - 1,1 Gew.-%, mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon.

Weitere bevorzugte erfindungsgemäße Zusammensetzungen enthalten, jeweils bezogen auf das Gesamtgewicht der Zusammensetzung, eine Mischung aus Dikaliummonocetylphosphat und Kaliumdicetylphosphat in einer Gesamtmenge von 0,5 - 2,0 Gew.-%, bevorzugt 0,8 - 1,8 Gew.-%, besonders bevorzugt 1,2 - 1,5 Gew.-%, weiterhin in einer Gesamtmenge von 0,3 - 1,7 Gew.-%, bevorzugt 0,6 - 1,4 Gew.-%, besonders bevorzugt 0,9 - 1,1 Gew.-%, mindestens eine Substanz, ausgewählt aus gehärteten Palmölglyceriden.

Eine erfindungsgemäß besonders bevorzugtes O/W-Emulgatorsystem, umfassend ein Gemisch aus Dikaliummonocetylphosphat und Kaliumdicetylphosphat mit gehärteten Palmölglyceriden, ist als Handelsprodukt "Emulsiphos 677660" (INCI: Potassium Cetyl Phosphate, Hydrogenated Palm Glycerides) von der Firma Symrise erhältlich.

Das O/W-Emulgatorsystem, bestehend aus mindestens einem Salz von $C_{12-20}$-Alkylphosphat als anionischem Öl-in-Wasser-Emulgator und mindestens einem $C_{14-20}$-Mono- oder Diacylglycerid, ist bevorzugt in einer Gesamtmenge von 0,5 Gew.-% bis 10 Gew.-%, besonders bevorzugt 1 bis 7 Gew.-%, insbesondere 2 - 4 Gew.-% und außerordentlich bevorzugt 2,5 - 3 Gew.-%, enthalten, jeweils bezogen auf das Gesamtgewicht der erfindungsgemäßen Zusammensetzung.

Die erfindungsgemäßen kosmetischen Zusammensetzungen enthalten kein Polysilicone-15. Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer, Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol® SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

Weitere bevorzugte erfindungsgemäße Zusammensetzungen enthalten a) 2-Cyano-3,3-phenyl-zimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Natriumsalz.

Weitere bevorzugte erfindungsgemäße Zusammensetzungen enthalten a) 2-Cyano-3,3-phenyl-zimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Argininsalz.

Weitere bevorzugte erfindungsgemäße Zusammensetzungen enthalten a) 2-Cyano-3,3-phenyl-zimtsäure-2-ethylhexylester, b) 4-(tert.-Butyl)-4'-methoxydibenzoylmethan, c) 3,3,5-Trimethylcyclohexylsalicylat und d) Dinatriumphenylbenzimidazoltetrasulfonat.

Weitere bevorzugte erfindungsgemäße Zusammensetzungen enthalten ein Gemisch aus Dikaliummonocetylphosphat und Kaliumdicetylphosphat mit gehärteten Palmölglyceriden, weiterhin a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat, d)i) 2-Phenylbenzimidazol-5-sulfonsäure-Argininsalz und d)ii) Dinatriumphenylbenzimidazoltetrasulfonat.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, jeweils bezogen auf das Gesamtgewicht der erfindungsgemäßen Zusammensetzung.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin

2-Cyano-3,3-phenylzimtsäure-2-ethylhexylester in einer Gesamtmenge von 0,5 - 15 Gew.-%, besonders bevorzugt 3 bis 13 Gew.-%, insbesondere 5 bis 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung.

Weitere bevorzugte erfindungsgemäße Zusammensetzungen enthalten die UV-Filter a), b), c) und d) in einem Gewichtsverhältnis zueinander von a) : b) : c) : d) wie (3 - 3,5) : (1 - 1,5) : (2 - 2,5) : (0,8 - 1,2).

Weitere bevorzugte erfindungsgemäße Zusammensetzungen enthalten die UV-Filter a), b), c) und d) in einem Gewichtsverhältnis zueinander von a) : b) : c) : d) wie (3,2 - 3,4) : (1,2 - 1,4) : (2,2 - 2,4) : (0,9 - 1,1 ), bevorzugt 3,3 : 1,3 : 2,3 : 1.

Weitere bevorzugte erfindungsgemäße Zusammensetzungen enthalten ein Gemisch aus Dikaliummonocetylphosphat und Kaliumdicetylphosphat mit gehärteten Palmölglyceriden und weiterhin 3,3,5-Trimethylcyclohexylsalicylat und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden, weiterhin a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat, d)i) 2-Phenylbenzimidazol-5-sulfonsäure-Argininsalz und d)ii) Dinatriumphenylbenzimidazoltetrasulfonat und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin 2-Cyano-3,3-phenylzimtsäure-2-ethylhexylester in einer Gesamtmenge von 0,5 - 15 Gew.-%, besonders bevorzugt 3 bis 13 Gew.-%, insbesondere 5 bis 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Weitere bevorzugte erfindungsgemäße Zusammensetzungen enthalten ein Gemisch aus Dikaliummonocetylphosphat und Kaliumdicetylphosphat mit gehärteten Palmölglyceriden in einer Gesamtmenge von 0,5 - 10 Gew.-%, besonders bevorzugt 1 - 7 Gew.-%, insbesondere 2 - 4 Gew.-% und außerordentlich bevorzugt 2,5 - 3 Gew.-%, weiterhin 2-Cyano-3,3-phenylzimtsäure-2-ethylhexylester in einer Gesamtmenge von 0,5 - 15 Gew.-%, besonders bevorzugt 3 - 13 Gew.-%, insbesondere 5 - 11 Gew.-% und außerordentlich bevorzugt 8 - 10 Gew.-%, 4-tert-Butyl-4'-methoxydibenzoylmethan in einer Gesamtmenge von 0,5 - 10 Gew.-%, bevorzugt 1 - 8 Gew.-%, besonders bevorzugt 2 - 5 Gew.-% und außerordentlich bevorzugt 3 - 4 Gew.-%, 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, das Argininsalz der 2-Phenylbenzimidazol-5-sulfonsäure in einer Gesamtmenge von 0,5 - 8 Gew.-%, bevorzugt 1 - 6 Gew.-%, besonders bevorzugt 1,5 - 4 Gew.-% und außerordentlich bevorzugt 2 - 3 Gew.-%, und Dinatriumphenylbenzimidazoltetrasulfonat in einer Gesamtmenge von 0,1 - 10 Gew.-%, bevorzugt 0,5 - 8 Gew.-%, besonders bevorzugt 0,8 - 5 Gew.-% und außerordentlich bevorzugt 0,9 - 2 Gew.-%, jeweils bezogen auf das Gesamtgewicht der erfindungsgemäßen Zusammensetzung, und weisen einen pH-Wert bei 20°C von 5,7 - 7,3, bevorzugt 6,0 - 6,7, besonders bevorzugt 6,2 bis 6,4, auf.

Um den Lichtschutzfaktor weiter zu erhöhen, können die erfindungsgemäßen Zusammensetzungen bevorzugt mindestens eine weitere organische und/oder anorganische UV-Filtersubstanz enthalten. s-Triazinderivate

Überraschend wurde festgestellt, dass mit öllöslichen organischen UV-Filtersubstanzen auf Basis von s-Triazinderivaten, die das nachfolgende Strukturmotiv

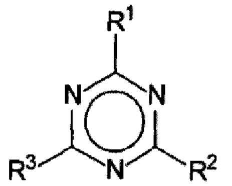

TRIAZIN-GRUND

aufweisen, teilweise geringere SPF-Werte und geringere Lagerstabilitäten erzielt werden konnten als beispielsweise mit 3,3,5-Trimethylcyclohexylsalicylat. In einer bevorzugten Ausführungsform sind die erfindungsgemäßen Zusammensetzungen daher frei von s-Triazinderivaten, die das oben stehende Strukturmotiv TRIAZIN-GRUND aufweisen.

s-Triazinderivate, die das oben stehende Strukturmotiv TRIAZIN-GRUND aufweisen, sind im Stand der Technik bekannt, beispielsweise aus EP 775698, EP 878469 und EP 1027881.

Hinsichtlich der $C_3$-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten $R^1$ $R^2$ und $R^3$ auf, während unsymmetrisch substituierte s-Triazinderivate demzufolge unterschiedliche Substituenten aufweisen, wodurch die $C_3$-Symmetrie zerstört wird. Im Sinne der vorliegenden Erfindung wird als "unsymmetrisch" stets unsymmetrisch hinsichtlich der $C_3$-Achse des Triazin-grundkörpers verstanden, es sei denn, etwas anderes wäre ausdrücklich erwähnt.

Hinsichtlich der $C_3$-Achse des Triazin-Grundkörpers symmetrische Triazinderivate sind beispielsweise solche der allgemeinen Formel TRIAZIN-GRUND mit $R^1 = R^2 = R^3$ = -NH-Phe-COOR, mit Phe = Phenyl-Rest, bei dem die Substituenten -NH und -COOR in para-Position zueinander stehen. Entsprechende symmetrische Triazinderivate sind 4,4',4"-(1,3,5-Triazin-2,4,6-triyltriimino)-tris-benzoesäure-tris(alkylester), insbesondere 4,4',4"-(1,3,5-Triazin-2,4,6-triyltrümino)-tris-benzoesäure-tris(2-ethylhexylester) [INCI: Ethylhexyl Triazone, vormals Octyl Triazone], das als Einzelsubstanz unter dem Namen UVINUL® T 150 vertrieben wird, aber auch in diversen kommerziellen UV-Filtermischungen erhältlich ist.

Unsymmetrische Triazinderivate sind beispielsweise solche, die in EP 775698 offenbart sind:

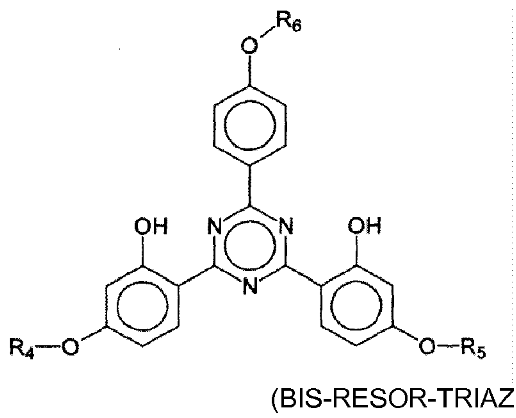

(BIS-RESOR-TRIAZIN)-I

und/oder

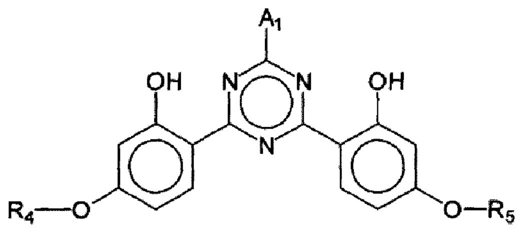

(BIS-RESOR-TRIAZIN)-II

$R_4$ und $R_5$ können aus der Gruppe der verzweigten und unverzweigten Alkylgruppen von 1 bis 18 Kohlenstoffatomen gewählt. Auch können die Alkylgruppen wiederum mit Silyloxygruppen substituiert sein.

$A_1$ stellt beispielsweise einen substituierten homo- oder heterocyclischen aromatischen Fünfring oder Sechsring dar.

In der allgemeinen Formel (BIS-RESOR-TRIAZIN)-I kann $R_6$ ein Wasserstoffatom oder eine verzweigte oder unverzweigte Alkylgruppe mit 1 - 10 Kohlenstoffatomen darstellen. Eine beispielhafte Verbindung der allgemeinen Formel (BIS-RESOR-TRIAZIN)-I, in der $R_4$ und $R_5$ jeweils eine 2-Ethylhexyl-Gruppe und $R_6$ eine Methylgruppe darstellen, ist 2,4-Bis-{[4-(2-ethyl-hexyloxy)-2-hydro-xy]-phenyl}-6-(4-methoxyphenyl)-1,3,5-triazin (INCI: Bis-Ethylhexyloxyphenol Methoxyphenyl Triazine), unter dem Handelsnamen Tinosorb® S von CIBA erhältlich.

Eine weitere beispielhafte unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit $R^1$ = $R^2$ -NH-Phe-COOR, mit Phe = Phenyl-Rest, bei dem die Substituenten -NH und -COOR in para-Position zueinander stehen, R = 2-Ethylhexyl und $R^3$ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein UV-A-Filter, ist unter der Handelsbezeichnung UVASORB HEB bei Sigma 3V erhältlich ist.

Weitere UV-Filtersubstanzen auf Basis von s-Triazin-Verbindungen sind:

- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb® K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,

- 2-[4,6-Bis(2,4-dimethylphenyl)-1,3,5-triazin-2-yl]-5-(ethylhexyloxy)phenol.

Um den Lichtschutzfaktor weiter zu erhöhen, können die erfindungsgemäßen Zusammensetzungen bevorzugt mindestens eine weitere organische und/oder anorganische UV-Filtersubstanz enthalten, die besonders bevorzugt verschieden ist von s-Triazinderivaten, die das oben stehende Strukturmotiv TRIAZIN-GRUND aufweisen.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Natriumsalz und sind frei von s-Triazinderivaten, die das oben stehende Strukturmotiv TRIAZIN-GRUND aufweisen.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) 2-Phenylbenzimidazol-5-sulfonsäure-Argininsalz und sind frei von s-Triazinderivaten, die das oben stehende Strukturmotiv TRIAZIN-GRUND aufweisen.

Weitere bevorzugte erfindungsgemäße Zusammensetzungen enthalten a) 2-Cyano-3,3-phenylzimtsäure-2-ethylhexylester, b) 4-(tert.-Butyl)-4'-methoxydibenzoylmethan, c) 3,3,5-Trimethylcyclohexylsalicylat und d) Dinatriumphenylbenzimidazoltetrasulfonat und sind frei von s-Triazinderivaten, die das oben stehende Strukturmotiv TRIAZIN-GRUND aufweisen.

Weitere bevorzugte öllösliche organische UV-Filtersubstanzen, die vorteilhaft in den erfindungsgemäßen Zusammensetzungen eingesetzt werden können, sind im Folgenden genannt.

Benzotriazole

Eine weitere bevorzugte UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel

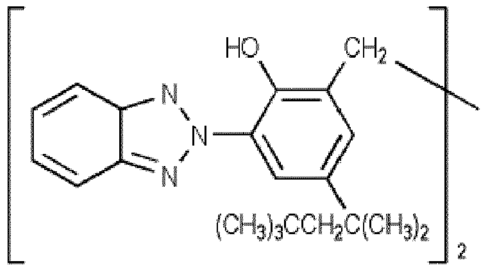

, unter der Handelsbezeichnung Tinosorb® M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (a), (b), (c) und (d) in den erfindungsgemäßen Zusammensetzungen eingesetzt werden, sind im Folgenden genannt:

- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor];
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolamin-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich);
- 4-Aminobenzoësäure-Derivate, bevorzugt 4-(Dimethylamino)-benzoësäure(2-ethylhexyl)ester, 4-(Dimethylamino)benzoësäureamylester;

- 2-Aminobenzoësäure-Derivate
- 4-Methoxyzimtsäure(2-ethylhexyl)ester,
- 4-Methoxyzimtsäurepropylester,
- 4-Methoxyzimtsäureisopentylester;
- Salicylsäure(2-ethylhexyl)ester (2-Ethylhexylsalicylat (= Octylsalicylat)),
- Salicylsäure(4-isopropylbenzyl)ester (4-Isopropylbenzylsalicylat),
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus von BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,
- an Polymere gebundene UV-Filter, die von Polysilicone-15 verschieden sind;
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb® K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0, besonders bevorzugt bei 0,5 - 0,75, außerordentlich bevorzugt bei 0,6 - 0,9.

In weiteren bevorzugten Kombinationen liegt das Gewichtsverhältnis von UV-B-Filter zu 1-(4-tert.-Butylphenyl)-3-(4'methoxyphenyl)propan-1,3-dion im Bereich von 0,5 bis 6, bevorzugt im Bereich von 0,6 bis 3, das molare Verhältnis liegt entsprechend im Bereich von 0,3 bis 3, bevorzugt im Bereich von 0,5 bis 1,5, besonders bevorzugt bei 0,5 - 0,75, außerordentlich bevorzugt bei 0,6 - 0,9. In weiteren bevorzugten Kombinationen liegt das Gewichtsverhältnis von Octocrylene zu 1-(4-tert.-Butylphenyl)-3-(4'methoxyphenyl)propan-1,3-dion im Bereich von 0,5 bis 6, bevorzugt im Bereich von 0,6 bis 3, das molare Verhältnis liegt entsprechend im Bereich von 0,3 bis 3, bevorzugt im Bereich von 0,5 bis 1,5, besonders bevorzugt bei 0,5 - 0,75, außerordentlich bevorzugt bei 0,6 - 0,9.

Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira.

Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Um die haptischen Eigenschaften der erfindungsgemäßen Zusammensetzungen weiter zu verbessern, enthalten bevorzugte erfindungsgemäße Zusammensetzungen Cetylalkohol und/oder Stearylalkohol und/oder Arachylalkohol und/oder Behenylalkohol. Besonders bevorzugte erfindungsgemäße Zusammensetzungen enthalten Cetylalkohol und/oder Stearylalkohol und/oder Arachylalkohol und/oder Behenylalkohol in einer Gesamtmenge von 0,5 - 3 Gew.-%, bevorzugt 1 - 2,5 Gew.-%, jeweils bezogen auf die gesamte Zusammensetzung.

Um die haptischen Eigenschaften und/oder den Lichtschutzfaktor der erfindungsgemäßen Zusammensetzungen weiter zu verbessern, können die erfindungsgemäßen Zusammensetzungen bevorzugt mindestens einen teilchenförmigen Feststoff, ausgewählt aus färbenden, farbgebenden, mattierenden oder glanzgebenden Pigmenten. Bevorzugte Pigmente dieser Art können anorganisch oder organisch sein. Weitere bevorzugte Pigmente weisen einen zahlenmittleren Teilchendurchmesser von 0,1 - 200 μm, bevorzugt 0,5 - 100 μm, besonders bevorzugt 1 - 50 μm und außerordentlich bevorzugt 2 - 30 μm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können.

Erfindungsgemäß bevorzugte Perlglanzpigmente sind ausgewählt aus natürlichen Perlglanzpigmenten, wie z. B. "Fischsilber" (Guanin/Hypoxanthin-Mischkristalle aus Fischschuppen) und "Perlmutt" (vermahlene Muschelschalen), monokristallinen Perlglanzpigmenten, wie z. B. Bismuthoxychlorid (BiOCl), und Schicht-Substrat Pigmenten, z. B. Glimmer / Metalloxid. Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere bevorzugt ist z. B. das unter der CIN 77163 aufgelistete Glanzpigment.

Es kann andererseits erfindungsgemäß bevorzugt sein, gänzlich auf ein Substrat wie Glimmer zu verzichten. Besonders bevorzugt sind Perlglanzpigmente, die unter der Verwendung von $SiO_2$ hergestellt werden. Solche Pigmente, die auch zusätzlich goniochromatische Effekte haben können, sind z. B. unter dem Handelsnamen Sicopearl Fantastico bei der Firma BASF erhältlich.

Weitere bevorzugte Pigmente sind ausgewählt aus farbigen und farblosen Pigmenten. Einige der im Folgenden genannten Pigmente dienen auch als UV-Absorber. Besonders bevorzugte farbige Pigmente sind ausgewählt aus den Eisenoxiden mit den Color Index-Nummern CI 77491 (Eisenoxid rot), CI 77492 (Eisenoxidhydrat gelb) und CI 77499 (Eisenoxid schwarz), aus CI 77891 (Titandioxid) und Ruß. Ein besonders bevorzugtes Pigment ist das Handelsprodukt SUNPMMA-S und SUNSIL Tin 30 von Sunjin Chemicals Co. mit einem zahlenmittleren Teilchendurchmesser von 5 - 10 μm bzw. 2 - 7 μm.

Besonders bevorzugte optionale anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere® LDP der Firma Merck KGaA. Bei diesem Produkt handelt es sich um sphärische Siliciumdioxid-Partikel, die mit Titandioxid und Eisenoxid beschichtet sind. Ronasphere® LDP weist einen zahlenmittleren Teilchendurchmesser von 4 - 7 μm auf.

Weiterhin bevorzugt sind anorganisch beschichtete Glimmerpigmente, die keinen Perlglanz aufweisen. Weitere bevorzugte optionale anorganisch beschichtete anorganische Pigmente sind Glimmerpigmente, die mit Titandioxid in verschiedenen Schichtdicken beschichtet sind, beispielsweise die Produkte der Timiron®-Serie von Rona/Merck KGaA, insbesondere Pigmente der Produktreihen Timiron® MP, Timiron® Super, Timiron® Starlight und Timiron® Silk. Die genannten Produkte weisen mittlere Teilchendurchmesser von 5 - 60 μm bzw. 10 - 60 μm bzw. 10 - 125 μm bzw. 5 - 25 μm auf. Ebenfalls bevorzugt sind Glimmerpartikel mit einer Beschichtung aus Titandioxid und Eisenoxid, z. B. die Handelsprodukte Timiron® MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona®-Serie. Weitere bevorzugte Pigmente sind mit Kieselgel beschichtete Glimmerpigmente, z. B. das Handelsprodukt Micronasphere® M. Weitere erfindungsgemäß bevorzugte Pigmente sind anorganisch beschichtete anorganische Pigmente, deren Beschichtung einen Anteil von 0,1 - 1 Gew.-% Zinnoxid aufweist.

Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Très BN® UHP 1106 von Carborundum), mit einem Gemisch aus Dimethylpolysiloxan und Silicagel (Simethicone) und Aluminiumoxidhydrat (Alumina) beschichtetes Titandioxid (Eusolex® T 2000 von Merck), mit Octylsilanol beschichtetes Titandioxid oder sphärische Polyalkylsesquisiloxan-Partikel (z. B. Aerosil® R972 von Evonik).

Weitere erfindungsgemäß besonders bevorzugte Zusammensetzungen enthalten mindestens ein färbendes, farbgebendes, mattierendes oder glanzgebendes Pigment in einer Gesamtmenge von 0,1 Gew.-% bis 30 Gew.-%, bevorzugt 0,5 bis 15 Gew.-%, besonders bevorzugt 1,0 bis 10 Gew.-% und außerordentlich bevorzugt 2 - 5 Gew.-%,

jeweils bezogen auf das Gesamtgewicht der erfindungsgemäßen Zusammensetzung.

Weitere bevorzugte optionale teilchenförmige Feststoffe sind die durch Hydrolyse und Kondensationsreaktionen von Methyltrimethoxysilane erhältlichen Polymethylsilsesquioxane, die sphärisch sein können (insbesondere Aerosil® R 972 und Aerosil® 200 V von Evonik Degussa), und deren Teilchengrößenverteilung durch die Herstellung gesteuert werden kann.

Die optionalen teilchenförmigen Feststoffe können sowohl einzeln als auch im Gemisch eingesetzt werden.

Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 -4 Gew.-%, jeweils bezogen auf das Gesamtgewicht der erfindungsgemäßen Zusammensetzung.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf:

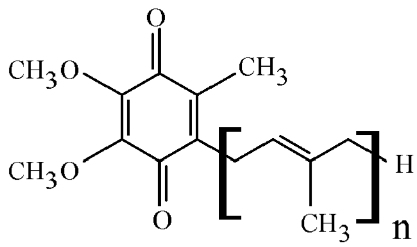

(UBI-I)

mit n = 6, 7, 8, 9 oder 10.

Besonders bevorzugt ist das Ubichinon der Formel (UBI-I) mit n = 10, auch bekannt als Coenzym Q10.

Erfindungsgemäß besonders bevorzugte Zusammensetzungen enthalten mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Die Herkunft der eingesetzten natürlichen Betainverbindung kann erfindungsgemäß durchaus synthetisch sein. Erfindungsgemäß bevorzugte natürliche Betainverbindungen sind natürlich vorkommende Verbindungen mit der Atomgruppierung $R_3N^+-CH_2-X-COO^-$ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain ($Me_3N^+-CH_2-COO^-$), Carnitin ($Me_3N^+-CH_2-CHOH-CH_2-COO^-$), jeweils mit Me = Methyl, und Ergothionein = 2-Mercapto-$N^\alpha N^\alpha N^\alpha$-trimethyl-L-histidinium-Betain der Formel (BETA-II)

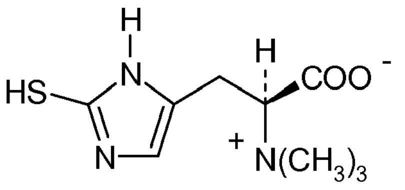

(BETA-II).

Weiterhin können auch Salze und/oder Ester der natürlichen Betainverbindungen erfindungsgemäß bevorzugt

eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden. Bevorzugte erfindungsgemäße Zusammensetzungen sind dadurch gekennzeichnet, dass sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Weitere erfindungsgemäß bevorzugte Zusammensetzungen sind dadurch gekennzeichnet, dass sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten $C_{8-30}$-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von $C_{6}$-$_{30}$-Fettsäuren mit $C_{2}$-$_{30}$-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol® CC), Mono,- Di- und Trifettsäureester von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter $C_{16-30}$-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder HydroxyGruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein. In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversionstemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare $C_8$-$C_{22}$-Fettalkohole, an $C_{12}$-$C_{22}$-Fettsäuren und an $C_8$-$C_{15}$-Alkylphenole, $C_{12}$-$C_{22}$-Fettsäuremono- und -diester von Anlagerungsprodukten von 1 bis 30 Mol Ethylenoxid an $C_3$-$C_6$-Polyole, insbesondere an Glycerin, Ethylenoxid- und

Polyglycerin-Anlagerungsprodukte an Methylglucosid-Fettsäureester, Fettsäurealkanolamide und Fettsäureglucamide, $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov® 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten $C_8$-$C_{22}$-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie lineare und verzweigte $C_8$-$C_{30}$-Fettsäuren und deren Na-, K-, Ammonium-, Ca-, Mg- und Zn-Salze.

Die erfindungsgemäßen Zusammensetzungen enthalten die Emulgatoren bevorzugt in einer Gesamtmenge von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel $R^1$ - O - $R^2$, in der $R^1$ eine primäre lineare Alkyl-, Alkenyl- oder Acylgruppe mit 20 - 30 C-Atomen, bevorzugt ein Behenyl-Rest, und $R^2$ Wasserstoff, eine Gruppe mit der Formel $-(C_nH_{2n}O)_x-H$ mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung $R^1$ - O - $R^2$ ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von $C_{16}$-$C_{30}$-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel $R^1$ - O - $R^2$ kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren. Weitere geeignete Zusatzstoffe sind Verdikkungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel®305, Simulgel® 600, Simulgel® NS und Simulgel® EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte $C_{3-6}$-Carbonsäure oder ihr Anhydrid sowie zu 2 - 20 % gewünschtenfalls substituierte Acrylsäureester von gesättigten $C_{10-30}$-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol® sowie Vinylpyrrolidon/Vinylester-Copolymere und Polyvinylpyrrolidone, die z. B. unter dem Warenzeichen Luviskol® (BASF) vertrieben werden.

Weitere bevorzugte Zusatzstoffe sind Lösungsmittel wie Ethanol, Isopropanol, Ethylenglycol, Propylenglycol, Propylenglykolmonoethylether, Glycerin und Diethylenglykol, Parfümöle, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, $N_2O$ Dimethylether, $CO_2$ und Luft.

In einer weiteren bevorzugten Ausführungsform liegen die erfindungsgemäßen Zusammensetzungen in einem Sprühspender oder Pumpspender verpackt vor.

Die nachfolgenden Beispiele sollen den Gegenstand der vorliegenden Anmeldung verdeutlichen, ohne ihn hierauf einzuschränken.

Beispiele:

Die Zahlenwerte in den Beispielen bedeuten Gewichtsprozente, bezogen auf das Gesamtgewicht der jeweiligen Zusammensetzungen.

Die Methode der Antioxidative Power (AP) ermöglicht die Bestimmung der antioxidativen Aktivität von aktiven Inhaltsstoffen in kosmetischen Produkten wie z.B. Pflanzenextrakten und Vitaminen. Die AP basiert auf der ESR (Elektronen Spin Resonanz) Spektroskopie, einer Methode, mit der freie Radikale quantitativ und qualitativ charakterisiert werden können. Damit impliziert die Methode auch die quantitative und qualitative Charakterisierung von Antioxidantien und Radikalfängern als den Antagonisten der freien Radikale.

Die Antioxidative Power ist eine zweidimensionale Größe, die sowohl die Kapazität eines Antioxidans, freie Radikale zu neutralisieren angibt, als auch seine Reaktivität. Die Kapazität wird anhand einer konzentrationsabhängigen Signalintensität ermittelt; die Reaktivität wird über das Erstellen von Reaktionskinetiken gemessen. So werden sowohl die Reaktionszeit ($t_r$) als auch die radikalreduzierende Kapazität erfasst und beide Größen fließen in die Berechnung der AP ein. Je schneller eine Substanz mit den freien Radikalen reagiert, desto niedriger ist ihre Reaktionszeit ($t_r$), desto höher ist ihre Antioxidative Power. Die Reaktionszeit ($t_r$) ist ein wichtiges Indiz für die Wirksamkeit einer Substanz und ihres Oxidationsgrades und ist ein Identifikationsmerkmal für ein Antioxidans. Die AP gibt an, wie viele Radikale von 1 mg Substanz in 1 Minute neutralisiert werden und wird in (rad/m*min) angegeben. Die AP Methode ist auf die Aktivität von Vitamin C (Ascorbinsäure) als Bezugspunkt standardisiert worden, so dass die AP in Antioxidative Units (AU) angegeben werden kann, wobei 1 AU der Aktivität von 1 ppm Vitamin C entspricht. Ein AP einer Substanz von 100 AU bedeutet, dass 1 mg der Substanz die gleiche Menge Radikale pro Zeiteinheit neutralisieren kann wie 0,1 pg Vitamin C.

Radical Skin Protection Factor (RSF)

Bei der RSF Methode werden die durch UV-Strahlung induzierten freien Radikale in der Haut gemessen. An Hand von experimentell bestimmten Gleichungen und mathematisch durchgeführten Kalibrierungen kann der Schutzfaktor (RSF), verursacht durch die topisch applizierten Produkte, bestimmt werden. Dabei gilt für die RSF-Bestimmung folgender Zusammenhang:

$$\text{RSF} = \text{Radikalmenge in ungeschützter Haut/Radikalmenge in geschützter Haut.}$$

Ähnlich dem SPF wird bei der Bestimmung des RSF die Wirkung der UV-Strahlung auf der Haut gemessen. Als Messgröße bzw. Indikator im Vergleich zwischen unbestrahlter und bestrahlter Haut wird nicht das als biologischer Endpunkte bezeichnete Erythem verwendet, sondern es wird die Menge und Art an UV generierten freien Radikalen in der Haut bestimmt. Die Menge an freien Radikalen ist proportional zur UV-Dosis und ihre Verteilung in den einzelnen Hautschichten (Epidermis, Dermis) wird maßgeblich von der Wellenlänge der eindringenden Photonen bestimmt. Die Bestimmung des RSF wird zweckmäßigerweise als ex vivo Methode an Hautbiopsien durchgeführt. Die Hautbiopsien werden mit einem Radikalindikator (Nitroxyl-Verbindung) markiert. Die durch die UV-Strahlung in der Haut generierten freien Radikale reagieren mit dem Radikalindikator, dessen Menge und Aktivität mit einem ESR-Spektrometer gemessen wird. Die Bildung der durch UVA und UVB induzierten freien Radikale ist ein Prozess, der sich in der epidermalen und dermalen Schicht der Haut abspielt. Die Wirkung der UVB-Strahlung beschränkt sich, bedingt durch die geringe Eindringtiefe, hauptsächlich auf die Epidermis. Der Radikalindikator ist hydrophil und weist demzufolge in der Dermis und Epidermis eine morphologisch bedingte Gleichverteilung auf. Der Radikalindikator selbst ist fotostabil und wird in wässriger Lösung nicht durch UV abgebaut.

Die in der Haut induzierten freien Radikale oxidieren das Molekül der Nitroxylverbindung und werden mit der ESR-Spektroskopie nachgewiesen. Über diesen Effekt kann die UV-schützende Wirkung (Radikalschutz) von UV-Filtern getestet werden. Während das UVA ausschließlich freie Radikale in der Haut generiert, kann das UVB (insbesondere das kurzwellige UVB) auch zu direkten Schäden an der Erbinformation (DNA) der Zelle führen. Dementsprechend ist die RSF-Methode besonders sensitiv gegenüber der Wirkung von UVA-Filtern.

Gleiche UV-Dosis für beide Messungen vorausgesetzt. Es wird ein Faktor angegeben, um den die generierte Radikalmenge bei Benutzung eines Sonnenschutzproduktes verringert wird.

Ebenfalls kann für eine gleiche vorgegebene induzierte Radikalmenge in beiden Fällen (ungeschützt versus geschützt) der Faktor angegeben werden, um den die Verweildauer unter UV-Strahlung für geschützte Haut erhöht werden kann. Eine direkte Bezugnahme zum SPF (Sonnenschutzfaktor) ist damit gegeben.

Hautmodell

Das verwendete Hautmodell basiert auf der Verwendung von Schweinehaut von Schlachttieren. Das Schweinehautmodell ist als dasjenige anerkannt, das der menschlichen Haut am ähnlichsten ist. Die Schweinehaut wurde

unmittelbar nach der Schlachtung (innerhalb von 4 Stunden) für die Hautuntersuchungen präpariert. Bis zur Messung wurden die Schweinehautstreifen (>1 cm x 1 cm) auf einer PBS-Lösung getränkten Gaze im Kühlschrank gelagert. Das Alter der Schlachttiere liegt einheitlich bei ca. 6 Monaten, so dass große altersbedingte Hautunterschiede nicht zu erwarten sind und bisher nicht beobachtet wurden.

Die Durchführung des Testverfahrens ist im Wesentlichen gekennzeichnet durch zwei Prozesse:

1. Einwirkung des Radikalindikators von der dermalen (5 min.) und der Probe der epidermalen (15 min.) Seite der Haut.
2. Die UV-Bestrahlung der Haut mit variablen Zeiten (entsprechend Versuchsprotokoll) von der epidermalen Seite und anschließender unmittelbarer Messung der induzierten freien Radikale mit der ESR-Spektroskopie.

Aus dem 1 $cm^2$ Hautareal wird eine 4 mm Ø große Biopsie ausgestanzt und auf einer ESR-Gewebezelle montiert. Danach erfolgt eine erste ESR-Messung (0-Messung). Die Biopsie wird mit zunehmenden UV-Dosen (UV-Bestrahlungszeiten) bestrahlt. Nach jeder Messung erfolgt eine Messung der Radikalintensität.

Die Bestrahlung erfolgt mit einem Sonnensimulator der Firma Hönle AG, Modell SOL2, bei einer Bestrahlungsstärke von E(UVA + UVB) = 23,2 mW/$cm^2$. Die einzelnen Bestrahlungsintensitäten betragen 22,0 mW/$cm^2$ für UVA und 1,2 mW/$cm^2$ für UVB. Das E(UVA)/E(UVB)-Verhältnis ist 22/2,1 = 18,3. Dieses Verhältnis entspricht der COLIPA-Anforderung, die sich nach den Sonnenintensitäts-Verhältnissen für die einzelnen Spektralbereiche richtet.

Es wurde folgende Rezeptur hergestellt (Mengenangaben in Gew.-%):

| | Creme A (erfindungsgemäß |
|---|---|
| Mischung aus Dikaliummonocetylphosphat und Kaliumdicetylphosphat (ex Emulsiphos 677660) | 1,5 |
| Hydrogenated Palm Glycerides (ex Emulsiphos 677660) | 0,9 |
| Lanette 22 | 1 |
| Cetearyl Alcohol | - |
| SymMollient S | 1 |
| PCL Solid 660086 | - |
| Bienenwachs | - |
| 2-Ethylhexyl-3,5,5-trimethylhexanoat | 3,5 |
| Octansäure-4-methyl-2-pentylbutylester | 2 |
| Cetiol B | - |
| Myritol 318 | - |
| Safloröl raffiniert | - |
| 3,3,5-Trimethylcyclohexylsalicylat | 7 |
| Octocrylene | 10 |
| Butylmethoxydibenzoylmethan | 4 |
| Parsol SLX | - |
| Neo Heliopan Hydro | 2 |
| Neo Heliopan AP | 1 |
| L-Arginin | 1 |
| Natriumhydroxid Perlen | 0,24 |
| AMP-ULTRA PC 2000 | - |
| Cosmedia Silc | 2 |
| Carbopol ETD 2020 | 0,2 |
| Xanthan | 0,2 |

(fortgesetzt)

| | Creme A (erfindungsgemäß |
|---|---|
| Aristoflex AVC | 0,6 |
| Cosmedia SP | - |
| Olivem 1000 | - |
| Tetranatrium-EDTA (Pulver) | 0,1 |
| Ethylparaben | 0,2 |
| Methylparaben | 0,2 |
| Phenoxyethanol | 0,4 |
| RonaCare AP | 0,05 |
| Lipochroman-6 | 0,01 |
| Vitamin E Acetat | 0,5 |
| Controx KS C | 0,05 |
| Hexandiol-1,6 | 4 |
| Glycerin | 5 |
| Parfum | 0,4 |
| Wasser vollentsalzt | ad 100 |

Liste der verwendeten Rohstoffe

| Handelsname | INCI | Hersteller |
|---|---|---|
| AMP-ULTRA PC 2000 | Aminomethyl propanol (2-Amino-2-methylpropanol), 95 Gew.-% (und 5 Gew.-% Wasser) | Dow (Angus Chemie) |
| Aristoflex AVC | AMMONIUM ACRYLOYLDIMETHYLTAURATE/VP COPOLYMER, T-BUTYL ALCOHOL | Clariant |
| Carbopol ETD 2020 | Acrylates/C10-30 Alkyl Acrylate Crosspolymer | Lubrizol |
| Cetiol B | DIBUTYL ADIPATE | Cognis |
| Controx KS | Tocopherol, Hydrogenated Palm Glycerides Citrate | Cognis |
| Cosmedia Silc | Silica (spherical) | Cognis |
| Cosmedia SP | Sodium Polyacrylate | Cognis |
| Emulsiphos 677660 | Potassium Cetyl Phosphate, Hydrogenated Palm Glycerides | Symrise |
| Lanette 22 | Behenyl alcohol (C22-Alkohol: 70-80 Gew.-%, C20-Alkohol: 10-20 Gew.-%, C18-Alkohol: 5-15 Gew.-%, C16-Alkohol: 0-0,5 Gew.-%, C24-Alkohol: 0-1 Gew.-%) | BASF/ Cognis |
| Lipochroman-6 | Dimethylmethoxy Chromanol | Lipotec |
| RonaCare AP | Bis-Ethylhexyl Hydroxydimethoxy Benzylmalonate | Merck KGaA |
| Myritol 318 | Caprylic/Capric Triglyceride | BASF/ Cognis |
| Neo Heliopan AP | Disodium Phenyl Dibenzimidazole Tetrasulfonate | Symrise |
| Neo Heliopan Hydro | Phenylbenzimidazol sulfonic acid | Symrise |
| Olivem 1000 | Cetearyl Olivate, Sorbitan Olivate | Erbslöh |

(fortgesetzt)

| Handelsname | INCI | Hersteller |
|---|---|---|
| PCL Solid 660086 | STEARYLHEPTANOAT + STEARYLOCTANOAT (STEARYL HEPTANOATE, STEARYL CAPRYLATE) | Symrise |
| SymMollient S | n-Hexadecyl-n-nonanoat + n-Octadecyl-n-nonanoat (Cetearyl Nonanoate) | Symrise |

Es wurde folgender Wert für die "Antioxidative Power" (AP) in Antioxidative Units (AU) ermittelt: 718 AU; tr = 0,19

Für die Skin Antioxidative Retention (SAR) nach Infrarotbestrahlung wurden folgende Werte ermittelt (n = 4 Proben):

Skin Antioxidative Retention (SAR) nach Infrarotbestrahlung

| Probe | Ohne Produkt | Sonnenschutz-formel | Positivkontrolle (Sonnenschutzfolie) |
|---|---|---|---|
| 1 | 91 | 101 | 101 |
| 2 | 90 | 99 | 96 |
| 3 | 88 | 95 | 94 |
| 4 | 82 | 90 | 94 |
| | | | |
| MW | 88 | 96 | 96 |
| SD | 4 | 5 | 3 |

**Patentansprüche**

1. Kosmetische Zusammensetzung, enthaltend

    a) 2-Cyano-3,3-phenylzimtsäure-2-ethylhexylester,
    b) 4-(tert.-Butyl)-4'-methoxydibenzoylmethan,
    c) 3,3,5-Trimethylcyclohexylsalicylat (Homosalate),
    d) mindestens einen wasserlöslichen organischen UV-Filter,
    e) mindestens eine Substanz, ausgewählt aus 6,7-disubstituierten 2,2-Dialkylchromanen oder -chromenen der allgemeinen Formeln (I) oder (II),

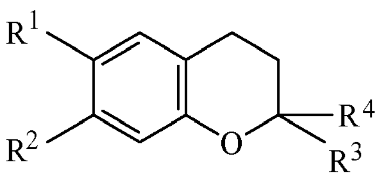

(I)

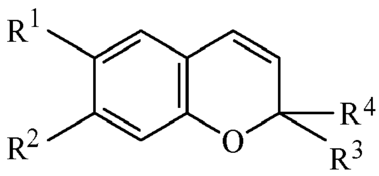

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere 3,4-Dihydro-7-methoxy-2,2-dimethyl-2H-1-benzopyran-6-ol, und wobei die Zusammensetzung kein Polysilicone-15 enthält.

2. Kosmetische Zusammensetzung gemäß Anspruch 1, **dadurch gekennzeichnet, dass** sie — bezogen auf ihr Gewicht - mindestens ein 6,7-disubstituiertes 2,2-Dialkylchroman oder -chromen der allgemeinen Formeln (I) oder (II) in einer Gesamtmenge von 0,001 - 2 Gew.-%, bevorzugt 0,005 - 1 Gew.-%, besonders bevorzugt 0,01 - 0,5 Gew.-

%, außerordentlich bevorzugt 0,05 - 0,2 Gew.-%, enthalten.

3. Kosmetische Zusammensetzung gemäß Anspruch 1 oder 2, **dadurch gekennzeichnet, dass** sie — bezogen auf ihr Gewicht - eine Mischung aus der oxidierten und der reduzierten Form von 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%, enthalten.

4. Kosmetische Zusammensetzung gemäß Anspruch 1, 2 oder 3, **dadurch gekennzeichnet, dass** der mindestens eine wasserlösliche organische UV-Filter mindestens ein Derivat der Benzimidazolsulfonsäure umfasst.

5. Kosmetische Zusammensetzung gemäß Anspruch 1, 2, 3 oder 4, **dadurch gekennzeichnet, dass** der mindestens eine wasserlösliche organische UV-Filter ausgewählt ist aus Phenylen-1,4-bis-(2-benzimidazyl)-3,3'-5,5'-tetrasulfonsäure, 2-Phenylbenzimidazol-5-sulfonsäure und/oder den Salzen dieser Säuren sowie aus Mischungen hiervon ausgewählt ist.

6. Kosmetische Zusammensetzung gemäß Anspruch 1, 2, 3, 4 oder 5, **dadurch gekennzeichnet, dass** das Salz des mindestens einen wasserlöslichen organischen UV-Filters, bevorzugt der Phenylen-1,4-bis-(2-benzimidazyl)-3,3'-5,5'-tetrasulfonsäure und/oder der 2-Phenylbenzimidazol-5-sulfonsäure, ausgewählt ist aus den Natrium-, Kalium-, Triethanolamin-, Lysin-, Arginin-, Histidin- und Ornithin-Salzen sowie den Salzen mit Amino-$C_1$-$C_6$-Alkanolen und/oder Amino-$C_1$-$C_6$-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) oder 2-Amino-2-(hydroxymethyl)propan-1,3-diol (TRIS), sowie aus Mischungen dieser Salze.

7. Kosmetische Zusammensetzung gemäß Anspruch 1, 2, 3, 4, 5 oder 6, **dadurch gekennzeichnet, dass** das/die Derivat/e der Benzimidazolsulfonsäure in einer Gesamtmenge von 0,5 bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sind.

8. Kosmetische Zusammensetzung gemäß Anspruch 1, 2, 3, 4, 5, 6 oder 7, **dadurch gekennzeichnet, dass** 2-Cyano-3,3-phenylzimtsäure-2-ethylhexylester in einer Gesamtmenge von 0,5 bis 15 Gew.-%, besonders bevorzugt 3 bis 13 Gew.-%, insbesondere 5 bis 11 Gew.-% und außerordentlich bevorzugt 8 - 10 Gew.-%, jeweils bezogen auf das Gewicht der gesamten Zusammensetzung, enthalten ist.

9. Kosmetische Zusammensetzung gemäß Anspruch 1, 2, 3, 4, 5, 6, 7 oder 8, **dadurch gekennzeichnet, dass** 4-(tert.-Butyl)-4'-methoxydibenzoylmethan in einer Gesamtmenge von 0,5 bis 10 Gew.-%, bevorzugt 1 bis 8 Gew.-%, besonders bevorzugt 2 bis 5 Gew.-% und außerordentlich bevorzugt 3 - 4 Gew.-%, jeweils bezogen auf das Gewicht der gesamten Zusammensetzung, enthalten ist.

10. Kosmetische Zusammensetzung gemäß Anspruch 1, 2, 3, 4, 5, 6, 7, 8 oder 9, **dadurch gekennzeichnet, dass** 3,3,5-Trimethylcyclohexylsalicylat in einer Gesamtmenge von 0,5 - 10 Gew.-%, bevorzugt 2 - 9 Gew.-%, besonders bevorzugt 4 - 8 Gew.-%, außerordentlich bevorzugt 5,5 - 7 Gew.-%, jeweils bezogen auf das Gewicht der gesamten Zusammensetzung, enthalten ist.

11. Kosmetische Zusammensetzung gemäß Anspruch Anspruch 1, 2, 3, 4, 5, 6, 7, 8, 9 oder 10, **gekennzeichnet durch** einen pH-Wert bei 20°C im Bereich von 5,7 - 7,3, bevorzugt im Bereich von 6,0 bis 6,7, besonders bevorzugt 6,2 bis 6,4.

12. Kosmetische Zusammensetzung gemäß Anspruch 1, 2, 3, 4, 5, 6, 7, 8, 9, 10 oder 11, **dadurch gekennzeichnet, dass** jeweils bezogen auf ihr Gesamtgewicht, 17 - 30 Gew.-%, bevorzugt 19 - 27 Gew.-%, besonders bevorzugt 21 - 25 Gew.-%, mindestens einer in der Ölphase oder der Wasserphase gelösten organischen UV-Filtersubstanz, darunter 2-Cyano-3,3-phenylzimtsäure-2-ethylhexylester, 4-(tert.-Butyl)-4'-methoxydibenzoylmethan und mindestens eine in der Wasserphase gelöste organische UV-Filtersubstanz, die ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren sowie aus Mischungen der genannten Sulfonsäurederivate, enthalten sind.

13. Kosmetische Zusammensetzung gemäß Anspruch 1, 2, 3, 4, 5, 6, 7, 8, 9, 10, 11 oder 12, **dadurch gekennzeichnet, dass** 2-Phenylbenzimidazol-5-sulfonsäure oder ein Salz hiervon in einer Gesamtmenge von 0,5 bis 8 Gew.-%, bevorzugt 1 bis 6 Gew.-%, besonders bevorzugt 1,5 bis 4 Gew.-% und außerordentlich bevorzugt 2 - 3 Gew.-%,

jeweils bezogen auf das Gesamtgewicht der erfindungsgemäßen Zusammensetzung, enthalten ist.

**14.** Kosmetische Zusammensetzung gemäß Anspruch 1, 2, 3, 4, 5, 6, 7, 8, 9, 10, 11, 12 oder 13, **dadurch gekennzeichnet, dass** Phenylen-1,4-bis-(2-benzimidazyl)-3,3'-5,5'-tetrasulfonsäure oder ein Salz hiervon in einer Gesamtmenge von 0,1 bis 10 Gew.-%, bevorzugt 0,5 bis 8 Gew.-%, besonders bevorzugt 0,8 bis 5 Gew.-% und außerordentlich bevorzugt 0,9 - 2 Gew.-%, jeweils bezogen auf das Gesamtgewicht der erfindungsgemäßen Zusammensetzung, enthalten ist.

**15.** Kosmetische Verwendung einer Zusammensetzung gemäß Anspruch 1, 2, 3, 4, 5, 6, 7, 8, 9, 10, 11, 12, 13 oder 14 zum Schutz der Haut oder des Haars vor UV-Strahlung und vor IR-Strahlung.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1591105 A1
- DE 102008025576
- EP 2266527 A2
- EP 709080 A2
- EP 775698 A
- EP 878469 A
- EP 1027881 A

### In der Beschreibung aufgeführte Nicht-Patentliteratur


- **J. KRUTMANN.** *Hautarzt,* 2003, vol. 54, 809-817